# EUROPEAN PATENT APPLICATION

(11) **EP 4 693 127 A1**
(43) Date of publication of application: **11.02.2026**
(21) Application number: 24784868.2
(22) Date of filing: 01.04.2024
(51) Int. Cl.: G06Q 10/06, G16H 50/30

(54) **PREDICTION SYSTEM, PREDICTION METHOD, AND PROGRAM**

(30) Priority: 06.04.2023 JP 2023062254
(71) Applicant: Shionogi & Co., Ltd., Osaka-shi, Osaka 541-0045 (JP)
(72) Inventor: IWAMOTO, Hiroki, Osaka-shi, Osaka 541-0045 (JP); MATSUMOTO NAKANO, Saki, Osaka-shi, Osaka 541-0045 (JP); KIGUCHI, Ryo, Osaka-shi, Osaka 541-0045 (JP)
(74) Representative: Vossius & Partner Patentanwälte Rechtsanwälte mbB
(86) International application number: PCT/JP2024/013429
(87) International publication number: WO 2024/210083

(57) **Abstract**

To accurately predict the level of future psychological stress of an object person. A prediction system (100) includes a first extraction unit (42) that extracts a similar worker group from a plurality of workers (W) on the basis of a degree of similarity of a working style to a working style of an object person (O) during a first period, a first model generation unit (43) that generates a first prediction model (57) by machine learning using first information, as learning data, the first information including at least working style information, measurement information, action record information, and psychological stress information of each worker (W) included in the similar worker group, and a first prediction unit (44) that predicts a level of psychological stress of the object person (O) by inputting input data including at least the working style information, the measurement information, and the action record information of the object person (O) to the first prediction model (57).

## Description

### [Technical Field]

The present invention relates to a prediction system that predicts the level of psychological stress of an object person, and the like.

### [Background Art]

Depression is a mental disorder with a high prevalence and a high recurrence rate. For this reason, depression tends to cause long-term illness, posing an economic loss to society. For this reason, there is an urgent need to prevent depression. One way to prevent depression is to recognize mental health problems at a pre-disease stage and take appropriate measures. For example, Patent Document 1 discloses technology for preventing and improving mental disorder by automatically calculating the risk of mental health problems and informing the individual, medical professionals, managers, and the like of the results and methods of prevention and improvement.

### [Prior Art Documents]

### [Patent Documents]

[Patent Document 1] Japanese Patent Application Publication No. 2022-176775

### [SUMMARY OF THE INVENTION]

### [TECHNICAL PROBLEMS TO BE SOLVED BY THE INVENTION]

In order to more effectively prevent depression, it is important to accurately predict the future state of mental health, that is, the level of future psychological stress.

An object of one aspect of the present invention is to realize a prediction system and the like that can accurately predict the level of future psychological stress of an object person.

### [MEANS FOR SOLVING THE PROBLEM]

In order to solve the above problem, a prediction system according to one aspect of the present invention includes a first extraction unit configured to extract a similar worker group from a plurality of workers on the basis of a degree of similarity of a working style to a working style of an object person during a first period, a first model generation unit configured to generate a first prediction model by machine learning using first information, as learning data, the first information including at least working style information indicating the working style of each worker included in the similar worker group, measurement information including an amount of activity and sleeping hours of each worker, action record information indicating records of the time of an action performed by each worker for each action type, and psychological stress information indicating a level of psychological stress of each worker, and a first prediction unit configured to predict a level of psychological stress of the object person from the first period onwards by inputting input data including at least the working style information, the measurement information, and the action record information of the object person to the first prediction model.

In order to solve the above problem, a prediction system according to one aspect of the present invention includes a second extraction unit configured to extract a similar worker group from a plurality of workers on the basis of a degree of similarity of an attendance status to an attendance status of an object person during a first period, a second model generation unit configured to generate a second prediction model by machine learning using second information, as learning data, the second information including at least attendance status information indicating an attendance status of each worker included in the similar worker group, measurement information including an amount of activity and sleeping hours of each worker, action record information indicating records of the time of an action performed by each worker for each action type, and psychological stress information indicating a level of psychological stress of each worker, and a second prediction unit configured to predict a level of psychological stress of the object person from the first period onwards by inputting input data including at least attendance status information, measurement information, and action record information of the object person to the second prediction model.

In order to solve the above problem, a prediction method according to one aspect of the present invention includes a first extraction step of extracting a similar worker group from a plurality of workers on the basis of a degree of similarity of a working style to a working style of an object person during a first period, a first model generation step of generating a first prediction model by machine learning using first information, as learning data, the first information including at least working style information indicating the working style of each worker included in the similar worker group, measurement information including an amount of activity and sleeping hours of each worker, action record information indicating records of the time of an action performed by each worker for each action type, and psychological stress information indicating a level of psychological stress of each worker, and a first prediction step of predicting a level of psychological stress of the object person from the first period onwards by inputting input data including at least the working style information, the measurement information, and the action record information of the object person to the first prediction model.

In order to solve the above problem, a prediction method according to one aspect of the present invention includes a second extraction step of extracting a similar worker group from a plurality of workers on the basis of a degree of similarity of an attendance status to an attendance status of an object person during a first period, a second model generation step of generating a second prediction model by machine learning using second information, as learning data, the second information including at least attendance status information indicating an attendance status of each worker included in the similar worker group, measurement information including an amount of activity and sleeping hours of each worker, action record information indicating records of the time of an action performed by each worker for each action type, and psychological stress information indicating a level of psychological stress of each worker, and a second prediction step of predicting a level of psychological stress of the object person from the first period onwards by inputting input data including at least attendance status information, measurement information, and action record information of the object person to the second prediction model.

The prediction system according to each aspect of the present invention may be implemented by a computer. In this case, a control program for the prediction system that causes the computer to operate as each part (software element) of the prediction system to implement the prediction system, and a computer-readable recording medium on which the control program is recorded also fall within the scope of the present invention.

### [EFFECT OF THE INVENTION]

According to one aspect of the present invention, it is possible to accurately predict the level of future psychological stress of an object person.

### [BRIEF DESCRIPTION OF THE DRAWINGS]

[Fig. 1]

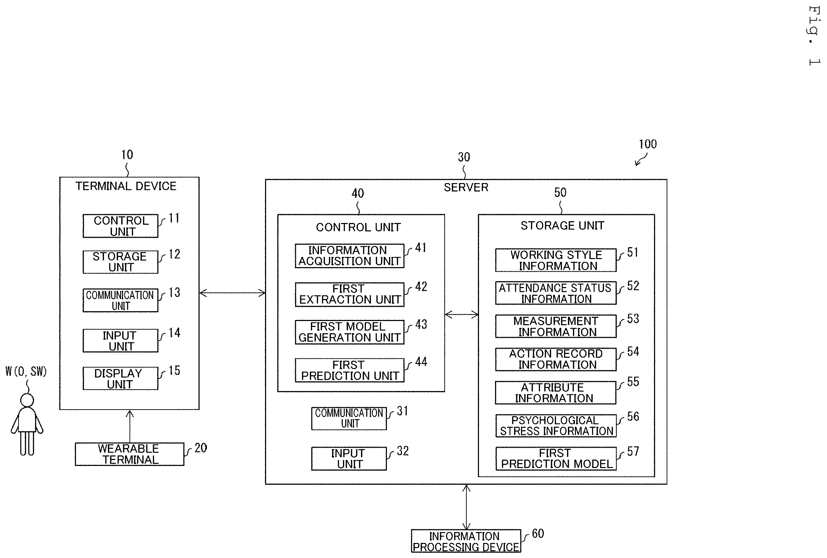

   Fig. 1 is a block diagram showing an example of the configuration of a prediction system according to Embodiment 1 of the present invention.
[Fig. 2]

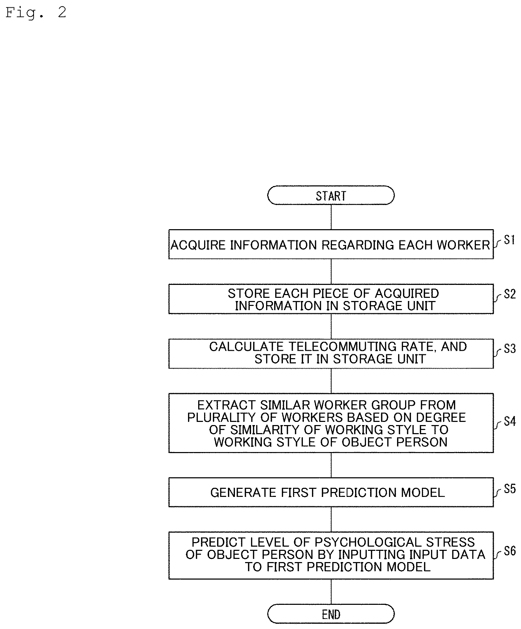

   Fig. 2 is a flowchart showing an example of a flow of processing performed by a server according to Embodiment 1 of the present invention.
[Fig. 3]

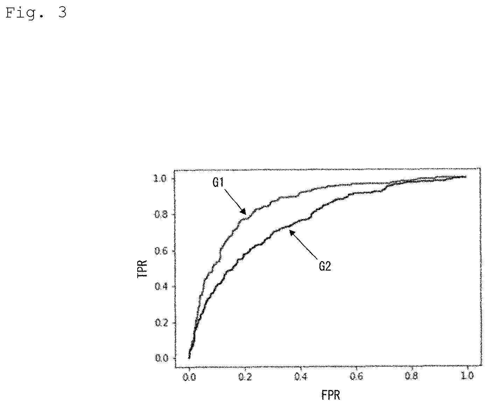

   Fig. 3 is a diagram showing ROC curves created on the basis of prediction results obtained when a prediction model of the related art is used and when a first prediction model generated by a method described in this embodiment is used, and an actual K6 score of each worker one week after prediction is performed.
[Fig. 4]

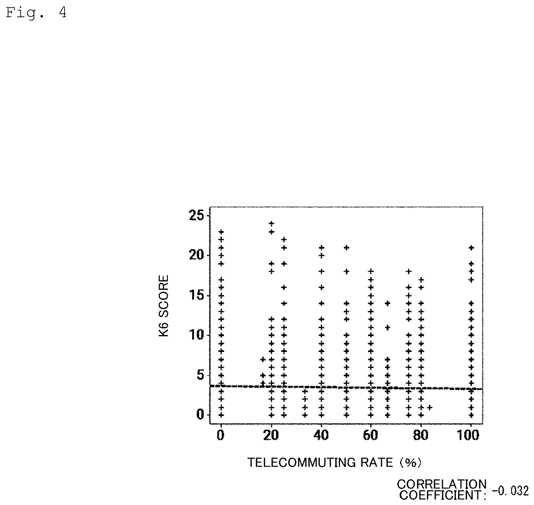

   Fig. 4 is a graph showing a relationship between a teleworking rate and the level of psychological stress.
[Fig. 5]

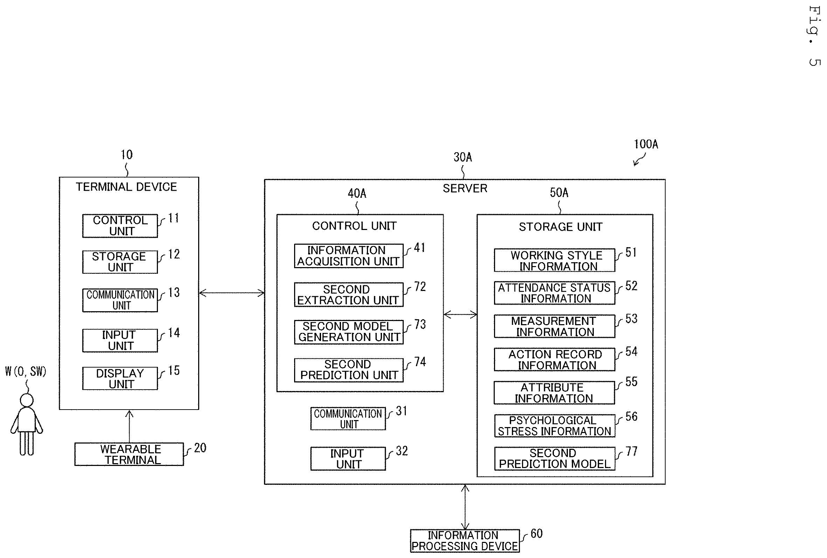

   Fig. 5 is a block diagram showing an example of the configuration of a prediction system according to Embodiment 2 of the present invention.
[Fig. 6]

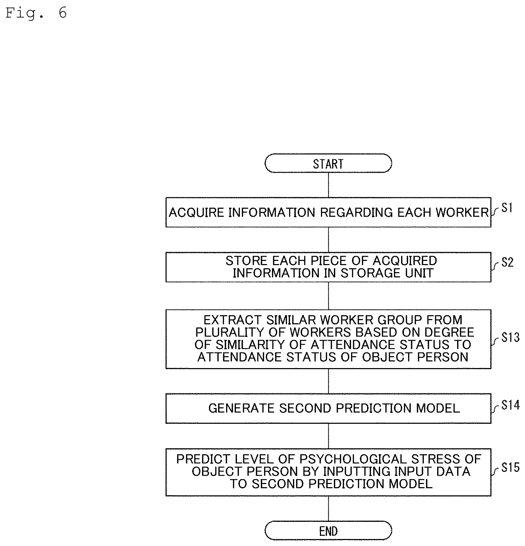

   Fig. 6 is a flowchart showing an example of a flow of processing performed by a server according to Embodiment 2 of the present invention.
[Fig. 7]

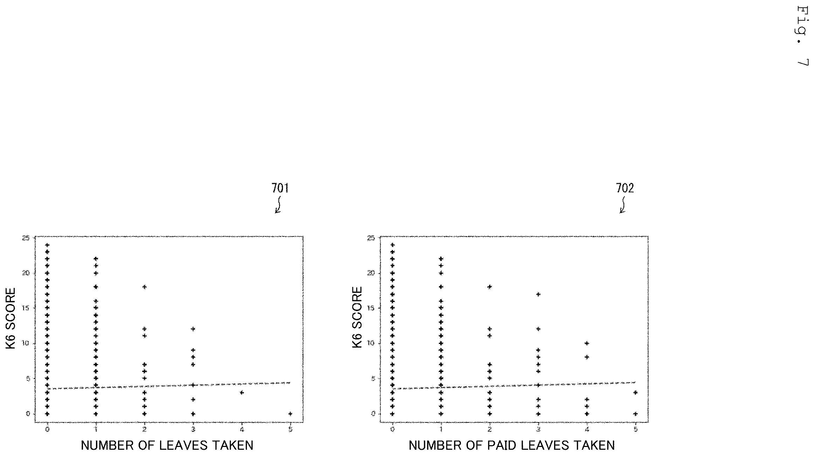

   Fig. 7 is graphs showing a relationship between the number of leaves taken or the number of annual paid leaves taken and the level of psychological stress.
[Fig. 8]

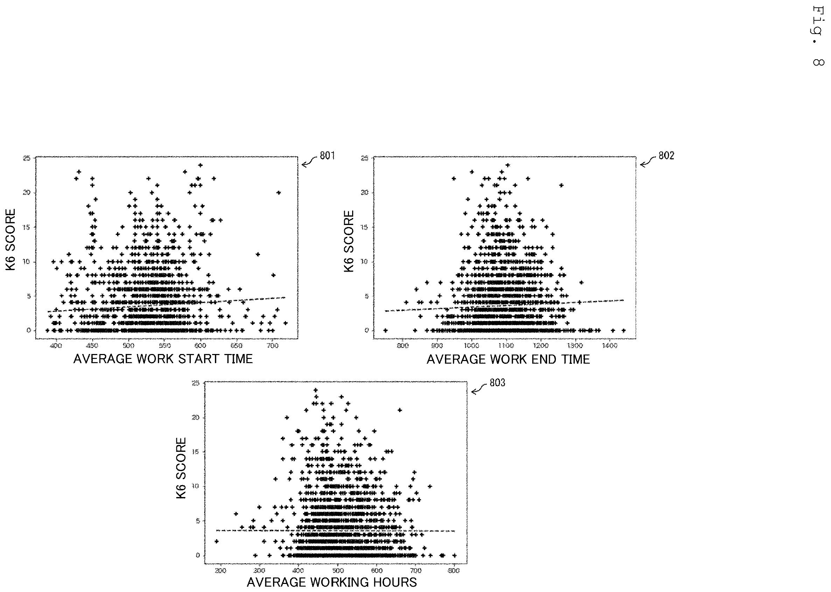

   Fig. 8 is graphs showing a relationship between average work start time, average work end time, or average working hours and the level of psychological stress.

### [MODE FOR CARRYING OUT THE INVENTION]

### [Embodiment 1]

One embodiment of the present invention will be described in detail below.

### (Configuration of prediction system 100)

A prediction system 100 in this embodiment is a system that predicts the level of future psychological stress of an object person on the basis of the object person's working style information, measurement information, and action record information. In this embodiment, it is assumed that all workers W who are subjects for prediction belong to the same company, and that the prediction system 100 has been introduced to that company. The prediction system 100 predicts the level of future psychological stress of workers, that is, laborers, and can also be referred to as a "laborer stress prediction system".

Fig. 1 is a block diagram showing an example of the configuration of the prediction system 100. As shown in Fig. 1, the prediction system 100 may include a terminal device 10 and a Wearable device 20 used by the worker W, a server 30, and an information processing device 60 owned by a person who manages attendance at a company to which the worker W belongs. Although Fig. 1 shows only one worker W, each of a plurality of workers W carries the terminal device 10 and the Wearable device 20 in the prediction system 100.

The prediction system 100 can predict the level of future psychological stress for each of the plurality of workers W. The workers W are workers who can select a desired working style from among a plurality of working styles. The plurality of working styles may include teleworking (working style in which work is performed from home). In this case, the worker W can select a desired working style from among at least teleworking and working at an office (working style in which a person goes to an office to work). In addition to the above, the worker W may also be able to select satellite work (working style in which work is performed at a location other than an office and home) as a working style.

The terminal device 10 may be a computer such as a smartphone or a tablet terminal. The terminal device 10 includes a control unit 11 that comprehensively controls each unit of the terminal device 10, a storage unit 12 that stores various data used by the terminal device 10, a communication unit 13 that enables the terminal device 10 to communicate with other devices, an input unit 14 that receives input operations for the terminal device 10, and a display unit 15 that displays various information.

In the terminal device 10, application software (hereinafter referred to as an application) for receiving an input of information indicating an action performed by the worker W and storing it in the storage unit 12 may be installed. The terminal device 10 may transmit information indicating the action performed by the worker W to the server 30 or the like via the communication unit 13. The terminal device 10 may receive, via the input unit 14 and through the above-described application, an input from the worker W regarding actions performed by the worker W and the time at which such actions were performed. Actions of the worker W may be classified into a plurality of items. For example, when actions of the worker W are classified into 16 items, the items may include "sleep", "meals/snacks", "bath", "work/study", "average watching time of media such as a TV or a DVD", and the like. Note that, in one aspect of the present invention, at least a part of information indicating an action pattern of the worker W may not be input by the worker W. For example, a sensor may detect that the worker W is watching media such as a TV or a DVD, and the sensor may calculate the "average watching time of media such as a TV or a DVD" on the basis of a detection result and output it to the server 30.

The terminal device 10 may have a function of acquiring information indicating the level of psychological stress of the worker W. For example, the terminal device 10 may have a function of performing a questionnaire to acquire information indicating the level of psychological stress. In this case, the terminal device 10 may receive an input of a response to the questionnaire via the input unit 14. An index indicating the level of psychological stress is not particularly limited, and conventionally known K6 score, PHQ-9, HAM-D, and The Brief Job Stress Questionnaire (57 items), and the like can be used. In this embodiment, an example of using the K6 score as an index indicating psychological stress will be described.

The Wearable device 20 is a device worn on the body of the worker W. The Wearable device 20 has a function of measuring data regarding the activity state (amount of activity) of the worker W. Here, the activity state may be the number of steps, burned calories, sleeping hours, a conversation time, a pulse rate, a skin temperature, the level of ultraviolet light irradiation, and the like. The Wearable device 20 may be configured to output information including the measured measurement data to the terminal device 10. The information including the measurement data may include the amount of activity and sleeping hours of the worker W. The Wearable device 20 may be, for example, a Wearable device worn on the head, neck, wrist, fingers, chest, abdomen, ankle, or the like of the worker W.

The server 30 may be a computer. The server 30 includes a control unit 40, a storage unit 50 that stores various data used by the server 30, a communication unit 31 that enables the server 30 to communicate with other devices, and an input unit 32 that receives input operations for the server 30.

The control unit 40 comprehensively controls each unit of the server 30. The control unit 40 includes an information acquisition unit 41, a first extraction unit 42, a first model generation unit 43, and a first prediction unit 44.

The information acquisition unit 41 acquires information regarding each worker W from the terminal device 10 or the information processing device 60 via the communication unit 31. Specifically, the information acquisition unit 41 acquires information indicating the working style of each worker W from the information processing device 60 via the communication unit 31. The information includes at least information on the date on which each worker W worked and information on the date on which each worker W teleworked. The information acquisition unit 41 stores, as working style information 51, the information indicating the working style of each worker W acquired from the information processing device 60 in the storage unit 50. In addition, the information acquisition unit 41 converts each data included in the information indicating the working style of each worker W acquired from the information processing device 60 into data for each predetermined period (for each week in this embodiment), and stores the information indicating each converted data in the storage unit 50 as the working style information 51. For example, the information acquisition unit 41 calculates, for each week, a teleworking rate, which is the ratio of the number of teleworking days to the total number of working days, on the basis of the information on the dates on which each worker W worked and the information on the dates on which they teleworked, which are included in the acquired working style information, and stores, in the storage unit 50, the teleworking rate information indicating the calculated teleworking rate as one piece of working style information 51.

The information acquisition unit 41 may also acquire information indicating the attendance status of each worker W from the information processing device 60. The attendance status information may include at least one among leave acquisition date and time, annual paid leave date and time, work start time, work end time, and working hours of each worker W. The information acquisition unit 41 stores, in the storage unit 50, the information indicating the attendance status of each worker W acquired from the information processing device 60 as attendance status information 52. The information acquisition unit 41 also converts each piece of data included in the acquired information indicating the attendance status of each worker W into data during a predetermined period (for each week in this embodiment), and stores, in the storage unit 50, information indicating each piece of converted data as attendance status information 52. For example, the information acquisition unit 41 may calculate the number of leaves taken for each week on the basis of leave acquisition date and time information included in the information indicating the attendance status of each worker W acquired from the information processing device 60. The information acquisition unit 41 may then store taken leave count information indicating the calculated number of leaves taken for each week in the storage unit 50 as one piece of attendance status information 52. Similarly, the information acquisition unit 41 may calculate, for each week, the number of annual paid leaves taken, average work start time, average work end time, average working hours, a standard deviation of work start time, a standard deviation of work end time, and a standard deviation of working hours. The information acquisition unit 41 may then store each piece of the calculated weekly data in the storage unit 50 as one piece of attendance status information 52.

The information acquisition unit 41 also acquires information including data on the activity state of the worker W measured by the Wearable device 20 from each of the terminal devices 10 owned by the worker W. The information acquisition unit 41 stores the information including the data on the activity state of the worker W acquired from the terminal device 10 in the storage unit 50 as measurement information 53. The information acquisition unit 41 also converts each piece of data included in the information including the data on the activity state of the worker W acquired from the terminal device 10 into data for each predetermined period (for each week in this embodiment), and stores information indicating each piece of converted data in the storage unit 50 as measurement information 53. For example, the information acquisition unit 41 may calculate an average value of the number of steps per day for each week on the basis of step count data information included in the information including the data on the activity state of the worker W acquired from the terminal device 10. Then, the information acquisition unit 41 may store, in the storage unit 50, information indicating the calculated average value of the number of steps per day for each week as one piece of measurement information 53. In addition, for example, the information acquisition unit 41 may calculate an average value of sleeping hours per day for each week on the basis of sleeping hours information included in the information acquired from the terminal device 10. Then, the information acquisition unit 41 may store, in the storage unit 50, information indicating the calculated average value of sleeping hours per day for each week as one piece of the measurement information 53.

The information acquisition unit 41 also acquires information indicating an action performed by each worker W. For example, the worker W may record the action performed by the worker W himself or herself in the terminal device 10 via the above-described application. In this case, the information acquisition unit 41 can acquire information indicating the action performed by each worker W from the terminal device 10 via the communication unit 31. However, a method by which the information acquisition unit 41 acquires the information indicating the action performed by each worker W is not limited thereto. For example, the information acquisition unit 41 may acquire information indicating the action performed by each worker W from a paper medium on which the worker W recorded his/her action record. In this case, a configuration may be adopted in which the action record recorded on the paper medium is input to the server 30 by, for example, a manager of the prediction system 100 via the input unit 32. Alternatively, the server 30 may be configured to be equipped with a known Optical Character Recognition (OCR) function and to directly read the action record recorded on the paper medium. The information acquisition unit 41 stores, in the storage unit 50, the acquired information indicating the action performed by each worker W as action record information 54.

The information acquisition unit 41 may also acquire information indicating the attributes of each worker W. The information indicating the attributes is information including, for example, gender, occupation, marital status, age, and the like. The information acquisition unit 41 stores the acquired information indicating the attributes in the storage unit 50 as attribute information 55. The information acquisition unit 41 may acquire, for example, information indicating the attributes input to each terminal device 10 by the worker W from each terminal device 10 via the communication unit 31. Alternatively, the information indicating the attributes may be input to the server 30 by, for example, the manager of the prediction system 100. In this case, a predetermined questionnaire for asking about the attribute information of the worker W is performed in advance for each worker W, and the manager or the like is only required to input attribute information 55 indicating the attributes of each worker W to the server 30 on the basis of responses to the questionnaire.

The information acquisition unit 41 also acquires information indicating the level of psychological stress of each worker W. The information acquisition unit 41 acquires the information indicating the level of the psychological stress of each worker W for every predetermined period (for each week in this embodiment), and stores the information indicating the level of psychological stress of each worker W for each week in the storage unit 50 as psychological stress information 56. For example, the information acquisition unit 41 can acquire the information indicating the level of psychological stress of each worker W from each terminal device 10 owned by each worker W via the communication unit 31. However, a method in which the information acquisition unit 41 acquires the level of psychological stress of each worker W is not limited thereto. For example, the information acquisition unit 41 may acquire the level of psychological stress of each worker W from a questionnaire sheet in which the worker W responds to a questionnaire performed to acquire the level of psychological stress of each worker W. In this case, for example, a configuration may be adopted in which the psychological stress information 56 created on the basis of the responses written on a paper medium is input to the server 30 via the input unit 32 by the manager of the prediction system 100. Alternatively, the server 30 may be equipped with an OCR function and may be configured to directly read the responses written on the questionnaire sheet.

The first extraction unit 42 extracts a similar worker group from among the plurality of workers W on the basis of the degree of similarity of a working style to the working style of a worker W (hereinafter, the worker W is referred to as an object person O) who is a target for predicting the level of psychological stress. The degree of similarity of the working style may be, for example, a difference between a teleworking rate, which is the ratio of the number of teleworking days to the total number of working days of the object person O, and the teleworking rate of the worker W. In this embodiment, a configuration will be described in which the first extraction unit 42 extracts a similar worker group on the basis of teleworking rate information indicating the teleworking rate. In this case, the first extraction unit 42 first reads, from the storage unit 50, a teleworking rate for each of the plurality of workers W during a period (hereinafter, the period will be referred to as a first period) between a point in time at which the prediction is performed (hereinafter, also referred to as the current point in time) and the point in time a first predetermined period before the current point in time (hereinafter, a first point in time). The length of the first period is not particularly limited, and may be, for example, a plurality of weeks. In this embodiment, the length of the first period will be described as 12 weeks. In this case, the first extraction unit 42 first reads, from the storage unit 50, a teleworking rate of each of the plurality of workers W between the current point in time and a point in time up to 12 weeks before the current point in time.

Based on the read-out teleworking rate, the first extraction unit 42 extracts, from the plurality of workers W, a similar worker group whose teleworking rates are similar to the teleworking rate of the object person O in the first period. For example, the first extraction unit 42 may extract a predetermined number of workers W as a similar worker group in order of their teleworking rates in the first period close to the teleworking rate of the object person O in the first period. In this case, the first extraction unit 42 may extract the similar worker group using a k-nearest neighbor method. Alternatively, the first extraction unit 42 may extract, as a similar worker group, workers W whose teleworking rates in the first period differ from the teleworking rate of the object person O in the first period within a predetermined range.

Here, as an example, a case where 20 workers W are extracted as a similar worker group is assumed. In this case, when the workers W ranked 16th to 25th in terms of the degree of similarity to the teleworking rate of the object person O in the first period have the same teleworking rate, the first extraction unit 42 first extracts, as a similar worker group, the workers W ranked 1st to 15th in terms of the degree of similarity to the teleworking rate of the object person O in the first period. The first extraction unit 42 may also randomly extract five workers W from the 10 workers W ranked 16th to 25th in terms of the degree of similarity to the teleworking rate of the object person O in the first period, and set the extracted workers as a similar worker group. As a result, a total of 20 similar workers are extracted. In the following, the workers W included in the similar worker group extracted by the first extraction unit 42 will be referred to as similar workers SW and described.

The first model generation unit 43 generates a prediction model (hereinafter referred to as a first prediction model 57) for predicting the level of psychological stress of the object person O. The first model generation unit 43 generates the first prediction model 57 by machine learning using first information including first explanatory variables and first objective variables to be described below as learning data. A machine learning model used to create the first prediction model 57 is not particularly limited, and for example, Xgboost, lightGBM, or the like can be used. Xgboost is an abbreviation for eXtreme Gradient Boosting, and is a method that combines ensemble learning referred to as gradient boosting and decision trees. lightGBM is a machine learning framework of gradient boosting based on a decision tree algorithm.

The first explanatory variables include at least the working style information 51, the measurement information 53, and the action record information 54 for each similar worker SW in the first period. The first explanatory variables may include a teleworking rate of the similar worker SW in the first period as the working style information 51. The first explanatory variables may include an amount of activity and sleeping hours of the similar worker SW in the first period as the measurement information 53. The amount of activity may include at least one of the number of steps, burned calories, sleeping hours, a conversation time, a pulse rate, a skin temperature, and the level of ultraviolet light irradiation. The first explanatory variables may include the number of times the similar worker SW eats lunch, outing information of the similar worker SW, and the like in the first period as the action record information 54. The first explanatory variables may include information (hereinafter referred to as leisure time information) on how each similar worker SW spends time that does not correspond to working hours and during which a desired action can be selected (hereinafter referred to as leisure time) as the action record information 54. The time during which a desired action can be selected may be a time obtained by subtracting working hours from the total time between wake-up and sleep of the similar worker SW. The leisure time information may include, for example, information regarding the length of leisure time.

The first explanatory variables may further include working style information 51, measurement information 53, and action record information 54 for the object person O during a period (hereinafter, the period will be referred to as a second period) between a point in time (hereinafter referred to as a second point in time) a second predetermined period before the point in time of the execution of prediction (that is, the current point in time) and the first point in time. In this embodiment, the first explanatory variables will be described as including the above information for the object person O during the second period. The length of the second period is shorter than the length of the first period, and in this embodiment, the length of the second period will be described as 7 weeks. Note that the length of the first period will be described as 12 weeks, as described above.

The first explanatory variables may include attendance status information 52 of each of the similar workers SW in the first period, and attendance status information 52 of the object person O during the second period. The first explanatory variables may also include attribute information 55 of each similar worker SW and the object person O.

The first objective variables include at least psychological stress information 56 indicating the level of psychological stress in the first period for each of the similar workers SW. When the first explanatory variables include working style information 51, measurement information 53, and action record information 54 for the object person O as in this embodiment, the first objective variables include psychological stress information 56 for the object person O during the second period.

The first model generation unit 43 reads each piece of information included in the first information (that is, the first explanatory variables and the first objective variables) from the storage unit 50, and generates the first prediction model 57 by machine learning using the first information configured with the read pieces of information as learning data. The first model generation unit 43 stores the generated first prediction model 57 in the storage unit 50.

The first prediction unit 44 predicts the level of psychological stress of the object person O from the first period onwards by inputting input data including at least the working style information 51, the measurement information 53, and the action record information 54 of the object person O to the first prediction model 57 generated by the first model generation unit 43. In this embodiment, the first prediction unit 44 predicts the level of psychological stress of the object person O from the first period onwards by inputting data to the first prediction model 57 as input data, the data including the above-described various information during a period (hereinafter, this period is referred to as a third period) from the second point in time to the point in time of the execution of prediction (that is, the current point in time). In this embodiment, as described above, since the length of the first period is 12 weeks and the length of the second period is 7 weeks, the length of the third period is five weeks. The first prediction unit 44 may predict the level of psychological stress of the object person O, for example, at a point in time several days to several weeks after the current point in time (that is, the latest point in time of the first period). In this embodiment, the first prediction unit 44 predicts the level of psychological stress of the object person O at a point in time one week after the current point in time.

The first prediction unit 44 may use the K6 score as an index indicating the level of psychological stress. In this case, the value of the K6 score may be set as the objective variable. As another example, the objective variables may be set to be classified into a plurality of classes on the basis of the value of the K6 score. For example, the objective variables may be set to be classified into a plurality of classes, such as class 0 for a K6 score less than 5, class 1 for a K6 score equal to or greater than 5 and less than 9, class 2 for a K6 score equal to or greater than 9 and less than 13, and class 3 for a K6 score equal to or greater than 13. As another example, the objective variables may be set to be classified into two classes, such as class 0 for a K6 score less than 5, and class 1 for a K6 score equal to or greater than 5. As an index indicating the level of psychological stress, other indices (for example, PHQ-9, HAM-D, and the like) may be used instead of the K6 score. In this embodiment, the K6 score is described as being used as an index indicating the level of psychological stress.

### (Processing performed by server 30)

A flow of processing performed by the server 30 will be described below with reference to Fig. 2. Fig. 2 is a flowchart showing an example of a flow of processing performed by the server 30.

First, the information acquisition unit 41 acquires information regarding each worker W (step S1). Specifically, the information acquisition unit 41 acquires, from the terminal device 10 or the information processing device 60, information indicating the working style of each worker W, information indicating the attendance status of each worker W, information including data on the activity state of each worker W which is measured by the Wearable device 20, information indicating actions performed by each worker W, information indicating the attributes of each worker W, and information indicating the level of psychological stress of each worker W.

The information acquisition unit 41 stores each piece of acquired information in the storage unit 50 (step S2). The information acquisition unit 41 also calculates a teleworking rate of each worker W for each week on the basis of information on the date on which each worker W worked and information on the date on which each worker W telecommuted, which are included in the acquired working style information, and stores teleworking rate information indicating the calculated teleworking rate in the storage unit 50 as one piece of working style information 51 (step S3).

Next, the first extraction unit 42 extracts a similar worker group from a plurality of workers W on the basis of the degree of similarity of a working style to the working style of an object person O (step S4, first extraction step). Specifically, the first extraction unit 42 reads out, from the storage unit 50, a teleworking rate for each of the plurality of workers W during a first period. Based on the read teleworking rates, the first extraction unit 42 extracts, from the plurality of workers W, a similar worker group whose teleworking rates are similar to the teleworking rate of the object person O in the first period.

Next, the first model generation unit 43 generates a first prediction model 57 for predicting the level of psychological stress of the object person O (step S5, first model generation step). Specifically, the first model generation unit 43 generates the first prediction model 57 by machine learning using first information as learning data, the first information including at least working style information 51, measurement information 53 of each worker W, action record information 54 of each worker W, and psychological stress information 56 of each worker W, during the first period, for each worker W included in the similar worker group extracted by the first extraction unit 42. The first model generation unit 43 reads each piece of information to be used as learning data from the storage unit 50.

Next, the first prediction unit 44 predicts the level of psychological stress of the object person O from the first period onwards by inputting input data to the first prediction model 57 generated by the first model generation unit 43 (step S6, first prediction step). Specifically, the first prediction unit 44 predicts the level of psychological stress of the object person O at a point in time one week after the current point in time by inputting input data including at least working style information 51, measurement information 53, and action record information 54 of the object person O during a third period to the first prediction model 57.

### (Effects of prediction system 100)

The inventors have found that, by using the first prediction model 57 generated by machine learning using, as learning data, first information including at least information on each similar worker SW included in a similar worker group whose working styles are highly similar to that of the object person O, it is possible to accurately predict the level of psychological stress of the object person O from the first period onwards, and have completed the prediction system 100 of this embodiment.

Here, description is given of results of a prediction accuracy test when a prediction model generated by a method of the related art was used, and when the first prediction model 57 generated by the method described in this embodiment was used. In this prediction accuracy test, 192 workers W working at the same company were tested as subjects.

When the prediction model of the related art was used, a prediction model was first generated by machine learning using, as training data, information including working style information 51, measurement information 53, action record information 54, and psychological stress information 56 of 192 workers W during a period from the point in time 12 weeks before the point in time of the execution of prediction to the point in time five weeks before the point in time of the execution of prediction. Next, the information including the working style information 51, the measurement information 53, and the action record information 54 of each worker W during a period from the point in time five weeks before the point in time of the execution of prediction to the point in time of the execution of prediction was input as input data to the generated prediction model, thereby predicting a K6 score one week after the point in time of the execution of prediction for each worker W.

Next, a case where a first prediction model 57 generated by the method described in this embodiment is used will be described. In this case, the first prediction model 57 is generated for each of the 192 workers W. Here, first, a method of generating the first prediction model 57 using one worker W among the 192 workers W as an object person O will be described. First, 20 similar workers SW were extracted as a similar worker group from the 191 workers W excluding the object person O in descending order of the degree of similarity of a teleworking rate to the teleworking rate of the object person O during a period (that is, a first period) from the point in time 12 weeks before the point in time of the execution of prediction to the point in time of the execution of prediction. Next, a first prediction model 57 for the object person O was generated by machine learning using, training data, first information including (1) working style information 51, measurement information 53, action record information 54, and psychological stress information 56 of the extracted 20 similar workers SW in the first period, and (2) working style information 51, measurement information 53, action record information 54, and psychological stress information 56 of the object person O during a period (that is, a second period) from the point in time 12 weeks before the point in time of the execution of prediction to the point in time five weeks before the point in time of the execution of prediction. For the other 191 workers W, first prediction models 57 were generated in the same manner. Next, information including working style information 51, measurement information 53, and action record information 54 for the corresponding worker W during a period from the point in time five weeks before the point in time of the execution of prediction to the point in time of the execution of prediction (that is, the third period) was input as input data to each of the first prediction models 57 generated for the 192 workers W, thereby predicting a K6 score for each worker W one week after the point in time at which the prediction was performed.

Fig. 3 is a diagram showing receiver operating characteristic (ROC) curves created on the basis of prediction results obtained when a prediction model of the related art was used and when the first prediction model 57 generated by the method described in this embodiment was used, and the actual K6 score (that is, the true value of the K6 score) of each worker W one week after the point in time at which prediction was performed. As shown in Fig. 3, it can be seen that an ROC curve G1 when the first prediction model 57 generated by the method described in this embodiment was used has a higher true positive rate (TPR) at most false positive rates (FPRs) and a higher prediction accuracy than an ROC curve G2 when the prediction model of the related art was used. When an area under the ROC curve (AUC) was calculated using each of the ROC curves shown in Fig. 3, the AUC was 0.760 when using the prediction model of the related art, whereas the AUC was 0.852 when using the first prediction model 57 generated by the method described in this embodiment. The criteria for evaluating AUC are described in "J.A.Swets "Measuring the accuracy of diagnostic systems" Science. 1988 Jun 3;240(4857):1285-93.doi: 10.1126/science.3287615." as follows: 0.50 to 0.60 is a "prediction at chance", 0.60 to 0.70 is a "poor prediction", 0.70 to 0.80 is a "fair prediction", 0.80 to 0.90 is a "good prediction", and 0.90 to 1.00 is an "excellent prediction", indicating that the prediction accuracy was high when the first prediction model 57 generated by the method described in this embodiment was used.

The reason why the prediction accuracy is high when using the first prediction model 57 generated by the method described in this embodiment is considered to be because the first prediction model 57 is generated using training data that includes information regarding each similar worker SW included in the similar worker group whose working styles are highly similar to that of the object person O.

Here, since the level of psychological stress is similar among workers with highly similar working styles, it is considered that the prediction accuracy of the level of psychological stress predicted using a prediction model generated using information of workers with highly similar working styles will increase. However, it should be noted that this idea is not appropriate. This will be described with reference to Fig. 4. Fig. 4 is a graph showing a relationship between a teleworking rate and the level of psychological stress. The graph shown in Fig. 4 is a graph in which the horizontal axis represents a teleworking rate during a predetermined period, and the vertical axis represents the level of psychological stress. The graph shown in Fig. 4 is a graph that is created using the data of 192 workers W as subjects in the above-described prediction accuracy test. When calculated from the graph shown in Fig. 4, a correlation coefficient between the teleworking rate and the level of psychological stress is - 0.032, indicating that there is a small correlation between the teleworking rate and the level of psychological stress.

In the prediction system 100 of this embodiment, the server 30 may output the prediction result of the level of psychological stress of the object person O predicted by the first prediction unit 44 to the terminal device 10 owned by the object person O via the communication unit 31. The terminal device 10 may receive the prediction result of the level of psychological stress of the object person O from the server 30 via the communication unit 13, and display a display screen for notifying the object person O of the prediction result of the level of psychological stress on the display unit 15. Thereby, the object person O can recognize the level of his or her psychological stress from the first period onwards. In addition, the terminal device 10 may display a display screen on the display unit 15 for displaying an alert to the object person O when the predicted value of the level of psychological stress is greater than a predetermined value. Thereby, the object person O can recognize that his or her psychological stress will increase from the first period onwards.

In the prediction system 100 of this embodiment, the server 30 may output the prediction result of the level of psychological stress of the object person O predicted by the first prediction unit 44 via the communication unit 31 to an information processing device owned by a manager (for example, the superior of workers W) who manages workers W in an organization (for example, a department, a group, or the like) to which the workers W belong. Thereby, the manager can ascertain the number of workers W in the organization who are predicted to have a high level of psychological stress, and thus the manager can manage the organization appropriately.

In the prediction system 100 of this embodiment, the server 30 may output the prediction result of the level of psychological stress of the object person O predicted by the first prediction unit 44 via the communication unit 31 to an information processing device owned by a human resources manager who manages the human resources of a company to which a worker W belongs. Thereby, the human resources manager can appropriately decide on company policies based on the number of workers W predicted to have a high level of psychological stress in the company, evaluate the workers W, and the like.

In the prediction system 100 of this embodiment, the server 30 may output the prediction result of the level of psychological stress of the object person O predicted by the first prediction unit 44 via the communication unit 31 to an information processing device owned by an industrial physician of a company to which a worker W belongs. Thereby, the industrial physician can take appropriate measures, such as conducting an interview with a worker W who is predicted to have a high level of psychological stress.

In the prediction system 100 of this embodiment, when the first extraction unit 42 extracts a similar worker group, a plurality of similar workers SW whose teleworking rates during a first period are similar to a teleworking rate of an object person O during the first period are extracted, and the first prediction model 57 is generated using first information including working style information 51, measurement information 53, action record information 54, and psychological stress information 56 of each similar worker SW during the first period. That is, a period in which input data to be input to the first prediction model 57 was acquired is the same as a period in which data included in the first information used to generate the first prediction model 57 was acquired. Thereby, it is possible to reduce the influence of a difference in psychological stress caused by the environment (for example, season, age, and the like) when the data was acquired. As a result, it is possible to improve the prediction accuracy of the level of psychological stress. Note that, in the prediction system 100 according to one aspect of this embodiment, when the first extraction unit 42 extracts a similar worker group, the first extraction unit 42 may extract a similar worker SW whose teleworking rate during an arbitrary period other than the first period is similar to the teleworking rate of the object person O during the first period. The first model generation unit 43 may then generate a first prediction model 57 using first information including working style information 51, measurement information 53, action record information 54, and psychological stress information 56 of the similar worker SW in the arbitrary period. That is, the period during which the input data to be input to the first prediction model 57 is acquired may be different from the period during which the data included in the first information used to generate the first prediction model 57 is acquired. The length of the arbitrary period may be the same length as the first period, or may be a period with a different length from the first period.

In this embodiment, it has been described that all workers W belong to the same company, but the present invention is not limited thereto. The prediction system 100 according to one aspect of the present invention may be applied to a plurality of workers W who belong to different companies, or may be applied to a group of workers in which some workers W and some other workers W work for different companies, as long as the workers W can select a desired working style from among a plurality of working styles.

### [Embodiment 2]

Another embodiment of the present invention will be described below. For ease of description, members having the same functions as those described in the above embodiment are denoted by the same reference numerals, and their descriptions will not be repeated.

In the prediction system 100 of Embodiment 1, the level of psychological stress of the object person O is predicted using the first prediction model 57 generated by machine learning using the first information as learning data, the first information including various information of a similar worker group extracted on the basis of the degree of similarity of a working style to the working style of the object person O. On the other hand, in a prediction system 100A of this embodiment, the level of psychological stress of an object person O is predicted using a prediction model generated by machine learning using second information as learning data, the second information including various information of a similar worker group extracted on the basis of the degree of similarity of an attendance status to the attendance status of the object person O.

Fig. 5 is a block diagram showing an example of the configuration of the prediction system 100A. As shown in Fig. 5, the prediction system 100A includes a server 30A instead of the server 30 in Embodiment 1. The server 30A includes a control unit 40A and a storage unit 50A instead of the control unit 40 and the storage unit 50 in Embodiment 1. The control unit 40A includes a second extraction unit 72, a second model generation unit 73, and a second prediction unit 74 instead of the first extraction unit 42, the first model generation unit 43, and the first prediction unit 44 in Embodiment 1.

The second extraction unit 72 extracts a similar worker group from among a plurality of workers W on the basis of the degree of similarity of an attendance status to that of an object person O whose level of psychological stress is to be predicted. The attendance status may be at least one of the number of leaves taken, the number of annual paid leaves taken, average work start time, average work end time, and average working hours. In this case, the degree of similarity of an attendance status may be, for example, a difference between the number of leaves taken by the object person O during a first period and the number of leaves taken by a worker W during a predetermined period (however, a period of the same length as the first period). In this embodiment, a configuration in which the second extraction unit 72 extracts a similar worker group on the basis of taken leave count information indicating the number of leaves taken will be described. In this case, the second extraction unit 72 first reads, from the storage unit 50A, the number of leaves taken for each of the workers W during a period (in this embodiment, the period is referred to as a first period) from the point in time of the execution of prediction (that is, the current point in time) to the point in time three predetermined periods before the current point in time (hereinafter referred to as a third point in time).

Based on the read number of leaves taken, the second extraction unit 72 extracts, from the plurality of workers W, a similar worker group whose numbers of leaves taken are similar to the number of leaves taken by the object person O during the first period. For example, the second extraction unit 72 may extract a predetermined number of workers W as a similar worker group in order of the number of leaves taken during the first period that is close to the number of leaves taken by the object person O during the first period. In this case, the second extraction unit 72 may extract the similar worker group using a k-nearest neighbor method. Alternatively, the second extraction unit 72 may extract, as a similar worker group, workers W whose number of leaves taken during the first period differs from the number of leaves taken by the object person O during the first period within a predetermined range. In the following, the workers W included in the similar worker group extracted by the second extraction unit 72 will be referred to as similar workers SW and described.

The second model generation unit 73 generates a prediction model (hereinafter referred to as a second prediction model 77) for predicting the level of psychological stress of the object person O. The second model generation unit 73 generates the second prediction model 77 by machine learning using, as learning data, second information including second explanatory variables and second objective variables to be described below. A machine learning model used to create the second prediction model 77 is not particularly limited, and for example, Xgboost, lightGBM, or the like can be used.

The second explanatory variables include at least attendance status information 52, measurement information 53, and action record information 54 for each of the similar workers SW during the first period. The second explanatory variables may include, as the attendance status information 52, information indicating at least one of the number of leaves taken, the number of annual paid leaves taken, average work start time, average work end time, and average working hours of the similar worker SW during the first period. The second explanatory variable may include, as the measurement information 53, the amount of activity and sleeping hours of the similar worker SW during the first period. The amount of activity may include at least one of the number of steps, burned calories, sleeping hours, a conversation time, a pulse rate, a skin temperature, and the level of ultraviolet light irradiation. The second explanatory variables may include, as the action record information 54, the number of times the similar worker SW eats lunch, outing information of the similar worker SW, and the like during the first period. The second explanatory variables may include leisure time information of each similar worker SW as the action record information. The leisure time information may include, for example, information on the amount of leisure time.

The second explanatory variables may include attendance status information 52, measurement information 53, and action record information 54 for the object person O during a period (in this embodiment, this period is referred to as a second period) between a point in time (hereinafter referred to as a fourth point in time) before a fourth predetermined period from the point in time of the execution of prediction (that is, the current point in time) and the third point in time. In this embodiment, the second explanatory variables will be described as including the above information for object person O during the second period. The length of the second period is shorter than the length of the first period, and in this embodiment, the length of the second period will be described as 7 weeks. Note that the length of the first period will be described as 12 weeks, as described above.

The second explanatory variables may include working style information 51 of each of the similar workers SW during the first period, and working style information 51 of the object person O during the second period. The second explanatory variables may also include attribute information 55 of each of the similar workers SW and the object person O.

The second objective variables include at least psychological stress information 56 indicating the level of psychological stress during the first period for each of the similar workers SW. When the second explanatory variables include attendance status information 52, measurement information 53, and action record information 54 for the object person O as in this embodiment, the second objective variables include psychological stress information 56 of the object person O during the second period.

The second model generation unit 73 reads each piece of information included in the second information (that is, the second explanatory variables and the second objective variables) from the storage unit 50A, and generates a second prediction model 77 by machine learning using the second information configured with the read pieces of information as learning data. The second model generation unit 73 stores the generated second prediction model 77 in the storage unit 50A.

The second prediction unit 74 predicts the level of psychological stress of the object person O from the first period onwards by inputting input data including at least the attendance status information 52, the measurement information 53, and the action record information 54 of the object person O to the second prediction model 77 generated by the second model generation unit 73. In this embodiment, the second prediction unit 74 predicts the level of psychological stress of the object person O from the first period onwards by inputting data to the second prediction model 77 as input data, the data including the above-described various information during a period (in this embodiment, the period is referred to as a third period) from the second point in time to the point in time of the execution of prediction (that is, the current point in time). In this embodiment, as described above, since the length of the first period is 12 weeks and the length of the second period is 7 weeks, the length of the third period is 5 weeks. The second prediction unit 74 may predict the level of psychological stress of the object person O, for example, at a point in time several days to several weeks after the current point in time (that is, the latest point in time of the first period). In this embodiment, the second prediction unit 74 predicts the level of psychological stress of the object person O at a point in time one week after the current point in time.

### (Processing performed by server 30A)

A flow of processing performed by the server 30A will be described below with reference to Fig. 6. Fig. 6 is a flowchart showing an example of a flow of processing performed by the server 30A.

First, in the processing performed by the server 30A, steps S1 and S2 described in Embodiment 1 are performed.

Next, the second extraction unit 72 extracts a similar worker group from a plurality of workers W on the basis of the degree of similarity of an attendance status to the attendance status of the object person O (step S13, second extraction step). Specifically, the second extraction unit 72 reads, from the storage unit 50A, the number of leaves taken for each of the plurality of workers W during a first period. Based on the read number of leaves taken, the second extraction unit 72 extracts, from the plurality of workers W, a similar worker group whose numbers of leaves taken during the first period are similar to the number of leaves taken by the object person O during the first period.

Next, the second model generation unit 73 generates a second prediction model 77 for predicting the level of psychological stress of the object person O (step S14, second model generation step). Specifically, the second model generation unit 73 generates the second prediction model 77 by machine learning using second information as learning data, the second information including at least attendance status information 52 of each worker W, measurement information 53 of each worker W, action record information 54 of each worker W, and psychological stress information 56 of each worker W, during the first period, included in the similar worker group extracted by the second extraction unit 72. The second model generation unit 73 reads each piece of information to be used as learning data from the storage unit 50A.

Next, the second prediction unit 74 predicts the level of psychological stress of the object person O from the first period onwards by inputting input data to the second prediction model 77 generated by the second model generation unit 73 (step S15, second prediction step). Specifically, the second prediction unit 74 predicts the level of psychological stress of the object person O at a point in time one week after the current point in time by inputting, to the second prediction model 77, input data including at least the attendance status information 52, the measurement information 53, and the action record information 54 of the object person O during a third period.

### (Effects of prediction system 100A)

The inventors have found that, by using the second prediction model 77 generated by machine learning using, as learning data, second information including at least information on each similar worker SW included in a similar worker group whose attendance statuses are highly similar to that of the object person O, it is possible to accurately predict the level of psychological stress of the object person O from the first period onwards, and have completed the prediction system 100A of this embodiment.

Here, description is given of results of a prediction accuracy test when a prediction model generated by a method of the related art was used, and when the second prediction model 77 generated by the method described in this embodiment was used. In this prediction accuracy test, 192 workers W working at the same company were tested as subjects.

The prediction model of the related art is the prediction model of the related art described in Embodiment 1. As described in Embodiment 1, when the prediction model of the related art was used, the AUC was 0.760.

When the second prediction model 77 generated by the method described in this embodiment was used, the second prediction model 77 was generated by the same method as the method of generating the first prediction model 57 in the prediction accuracy test described in Embodiment 1, except that, when extracting a similar worker group, the number of leaves taken, the number of annual paid leave taken, average work start time, average work end time, or average working hours were used instead of using a teleworking rate.

When the second prediction model 77 generated by the method described in this embodiment was used, the AUC was calculated in the same manner as the prediction accuracy test described in Embodiment 1. As a result, when a similar worker group is extracted, the AUC was 0.852 when the number of leaves taken was used, the AUC was 0.838 when the number of annual paid leaves taken was used, the AUC was 0.829 when the average work start time was used, the AUC was 0.832 when the average work end time was used, and the AUC was 0.830 when the average working hours were used, indicating that the prediction accuracy was high when the second prediction model 77 generated by the method described in this embodiment was used.

The reason why the prediction accuracy is high when using the second prediction model 77 generated by the method described in this embodiment is considered to be because the second prediction model 77 is generated using training data that includes information regarding each similar worker SW included in the similar worker group whose attendance statuses are highly similar to that of the object person O.

Here, since the level of psychological stress is similar among workers with highly similar attendance statuses, it is considered that the prediction accuracy of the level of psychological stress will increase. However, it should be noted that this idea is not appropriate. This will be described with reference to Figs. 7 and 8. Fig. 7 is graphs showing a relationship between the number of leaves taken or the number of annual paid leave taken and the level of psychological stress. The graph indicated by reference numeral 701 in Fig. 7 is a graph in which the horizontal axis represents the number of leaves taken during a predetermined period and the vertical axis represents a K6 score, and the graph indicated by reference numeral 702 in Fig. 7 is a graph in which the horizontal axis represents the number of annual paid leave taken during a predetermined period and the vertical axis represents the value of a K6 score. Fig. 8 is graphs showing a relationship between average work start time, average work end time, or average working hours and the level of psychological stress. The graph indicated by reference numeral 801 in Fig. 8 is a graph in which the horizontal axis represents average work start time during a predetermined period and the vertical axis represents a K6 score. In the graph indicated by reference numeral 801, the average work start time is shown as a time elapsed from midnight. The unit of the numerical values on the horizontal axis of the graph indicated by reference numeral 801 is minutes. The graph indicated by reference numeral 802 in Fig. 8 is a graph in which the horizontal axis represents average work end time during a predetermined period and the vertical axis represents a K6 score. In the graph indicated by reference numeral 802, the average work end time is shown as a time elapsed from midnight. The unit of the numerical values on the horizontal axis of the graph indicated by reference numeral 802 is minutes. The graph indicated by reference numeral 803 in Fig. 8 is a graph in which the horizontal axis represents average working hours during a predetermined period and the vertical axis represents a K6 score. The unit of the numerical values on the horizontal axis of the graph indicated by reference numeral 803 is minutes. As shown in Figs. 7 and 8, it can be seen that a correlation with an attendance status and the K6 score is small. Specifically, a coefficient of determination R² of a regression equation of the graph indicated by reference numeral 701 in Fig. 7 was 2.82 × 10⁻⁴, and a correlation coefficient between the number of leaves taken and the K6 score was 0.017. Furthermore, a coefficient of determination R² of a regression equation of the graph indicated by reference numeral 702 in Fig. 7 was 4.31 × 10⁻⁴, and a correlation coefficient between the number of annual paid leaves taken and the K6 score was 0.021. Furthermore, a coefficient of determination R² of a regression equation of the graph indicated by reference numeral 801 in Fig. 8 was 4.08 × 10⁻³, and a correlation coefficient between the average work start time and the K6 score was 0.063. Furthermore, a coefficient of determination R² of a regression equation of the graph indicated by reference numeral 802 in Fig. 8 was 1.45 × 10⁻³, and a correlation coefficient between the average work end time and the K6 score was 0.038. Furthermore, a coefficient of determination R² of a regression equation of the graph indicated by reference numeral 803 in Fig. 8 was 5.50 × 10⁻⁶, and a correlation coefficient between the average working hours and the K6 score was -0.002.

In the prediction system 100A of this embodiment, when the second extraction unit 72 extracts a similar worker group, a plurality of similar workers SW whose number of leaves taken during a first period is similar to the number of leaves taken by an object person O during the first period are extracted, and a second prediction model 77 is generated using second information including attendance status information 52, measurement information 53, action record information 54, and psychological stress information 56 of each similar worker SW during the first period. That is, a period in which input data to be input to the second prediction model 77 was acquired is the same as a period in which data included in the second information used to generate the second prediction model 77 was acquired. Thereby, it is possible to reduce the influence of a difference in psychological stress caused by the environment (for example, season, age, and the like) when the data was acquired. As a result, it is possible to improve the prediction accuracy of the level of psychological stress. Note that, in the prediction system 100A according to one aspect of this embodiment, when the second extraction unit 72 extracts a similar worker group, the second extraction unit 72 may extract a similar worker SW whose number of leaves taken during an arbitrary period other than the first period is similar to the number of leaves taken by the object person O during the first period. The second model generation unit 73 may then generate a second prediction model 77 using second information including attendance status information 52, measurement information 53, action record information 54, and psychological stress information 56 of the similar worker SW during the arbitrary period. That is, the period during which the input data to be input to the second prediction model 77 is acquired may be different from the period during which the data included in the second information used to generate the second prediction model 77 is acquired. The length of the arbitrary period may be the same length as the second period, or may be a period with a different length from the second period.

### [Example of implementation by software]

The functions of the server 30 or the server 30A (hereinafter referred to as a "device") can be implemented by a program for causing a computer to function as the device, the program causing the computer to function as each control block of the device (particularly each part included in the control unit 40 or the control unit 40A).

In this case, the device includes a computer including at least one control device (for example, a processor) and at least one storage device (for example, a memory) as hardware for executing the program. The functions described in each of the above embodiments are implemented by executing the program using the control device and the storage device.

The above program may be recorded on one or a plurality of non-transitory computer-readable recording media. The recording media may or may not be provided in the device. In the latter case, the above program may be supplied to the device via any wired or wireless transmission medium.

In addition, some or all of the functions of the above control blocks can be implemented by a logic circuit. For example, an integrated circuit in which a logic circuit that functions as each of the above control blocks is formed is also included in the scope of the present invention. In addition, it is also possible to implement the functions of the above control blocks by, for example, a quantum computer.

The present invention is not limited to the above-described embodiments, and various modifications can be made within the scope of the claims. Embodiments obtained by appropriately combining the technical means disclosed in different embodiments are also included in the technical scope of the present invention.

### (Summary)

The prediction system according to aspect 1 of the present disclosure includes a first extraction unit that extracts a similar worker group from a plurality of workers on the basis of the similarity of their working style with the working style of an object person during a first period; a first model generation unit that generates a first prediction model by machine learning using first information as learning data, the first information including at least: working style information indicating the working style of each worker included in the similar worker group, measurement information including the amount of activity and sleeping hours of each worker, action record information that records the time of action performed by each worker by action type, and psychological stress information indicating the level of psychological stress of each worker; and a first prediction unit that predicts the level of psychological stress of the object person from the first period onwards by inputting input data including at least the working style information, the measurement information, and the action record information of the object person into the first prediction model.

In the prediction system according to aspect 2 of the present disclosure, in aspect 1 above, wherein the plurality of working styles includes at least teleworking, and the first extraction unit may extract the similar worker group on the basis of teleworking rate information indicating the proportion of teleworking days to the total number of working days.

In the prediction system according to aspect 3 of the present disclosure, in aspect 1 or 2 above, the first model generation unit may use the first information, including the working style information, the measurement information, the action record information, and the psychological stress information of each of the workers during the first period, as the learning data.

The prediction system according to aspect 4 of the present disclosure includes a second extraction unit that extracts a similar worker group from a plurality of workers on the basis of the similarity of their attendance status with the attendance status of an object person during a first period; a second model generation unit that generates a second prediction model by machine learning using second information as learning data, the second information including at least: attendance status information indicating the attendance status of each worker included in the similar worker group, measurement information including the amount of activity and sleeping hours of each worker, action record information that records the time of action performed by each worker by action type, and psychological stress information indicating the level of psychological stress of each worker; and a second prediction unit that predicts the level of psychological stress of the object person from the first period onwards by inputting input data including at least the attendance status information, the measurement information, and the action record information of the object person into the second prediction model.

In the prediction system according to aspect 5 of the present disclosure, in aspect 4 above, the attendance status information may be information indicating at least one of the number of leaves taken, the number of annual paid leaves taken, the average work start time, the average work end time, and the average working hours.

In the prediction system according to aspect 6 of the present disclosure, in aspect 4 or 5 above, the second model generation unit may use the second information including the attendance status information, the measurement information, the action record information, and the psychological stress information of each worker during the first period as the learning data.

In the prediction system according to aspect 7 of the present disclosure, in any one of aspects 1 to 6 above, the action record information may include information on how each of the workers spends time that is not working hours and during which the desired action can be selected.

A prediction method according to aspect 8 of the present disclosure includes a first extraction step of extracting a similar worker group from a plurality of workers on the basis of the similarity of their working styles with the working style of an object person during a first period; a first model generation step of generating a first prediction model by machine learning using first information as learning data, the first information including at least: working style information indicating the working style of each worker included in the similar worker group, measurement information including the amount of activity and sleeping hours of each worker, action record information recording the time of action performed by each worker for each action type, and psychological stress information indicating the level of psychological stress of each worker; and a first prediction step of predicting the level of psychological stress of the object person from the first period onwards by inputting input data including at least the working style information, the measurement information, and the action record information of the object person into the first prediction model.

A prediction method according to aspect 9 of the present disclosure includes a second extraction step of extracting a similar worker group from a plurality of workers on the basis of the similarity of their attendance status with the attendance status of an object person during a first period; a second model generation step of generating a second prediction model by machine learning using second information as learning data, the second information including at least attendance status information indicating the attendance status of each worker included in the similar worker group, measurement information including the amount of activity and sleeping hours of each worker, action record information recording the time of action performed by each worker by action type, and psychological stress information indicating the level of psychological stress of each worker; and a second prediction step of predicting the level of psychological stress of the object person from the first period onwards by inputting input data including at least the attendance status information, the measurement information, and the action record information of the object person into the second prediction model.

A program according to aspect 10 of the present disclosure is a program for causing a computer to function as the prediction system of any one of aspects 1 to 3 above, and is a program for causing a computer to function as the first extraction unit, the first model generation unit, and the first prediction unit.

A program according to aspect 11 of the present disclosure is a program for causing a computer to function as the prediction system of any one of aspects 4 to 6 above, and is a program for causing a computer to function as the second extraction unit, the second model generation unit, and the second prediction unit.

### [REFERENCE SIGNS LIST]

- 42: First extraction unit
- 43: First model generation unit
- 44: First prediction unit
- 51: Working style information
- 52: Attendance status information
- 53: Measurement information
- 54: Action record information
- 56: Psychological stress information
- 57: First prediction model
- 72: Second extraction unit
- 73: Second model generation unit
- 74: Second prediction unit
- 77: Second prediction model
- 100, 100A: Prediction system

## Claims

1. A prediction system comprising:
a first extraction unit configured to extract a similar worker group from a plurality of workers on the basis of a degree of similarity of a working style to a working style of an object person during a first period;
a first model generation unit configured to generate a first prediction model by machine learning using first information, as learning data, the first information including at least working style information indicating the working style of each worker included in the similar worker group, measurement information including an amount of activity and sleeping hours of each worker, action record information indicating records of the time of an action performed by each worker for each action type, and psychological stress information indicating a level of psychological stress of each worker; and
a first prediction unit configured to predict a level of psychological stress of the object person from the first period onwards by inputting input data including at least the working style information, the measurement information, and the action record information of the object person to the first prediction model.

2. The prediction system according to claim 1, wherein
the plurality of working styles include at least teleworking, and
the first extraction unit extracts the similar worker group on the basis of teleworking rate information indicating a proportion of teleworking days to a total number of working days.

3. The prediction system according to claim 1, wherein the first model generation unit uses, as the learning data, the first information including the working style information, the measurement information, the action record information, and the psychological stress information of each worker during the first period.

4. A prediction system comprising:
a second extraction unit configured to extract a similar worker group from a plurality of workers on the basis of a degree of similarity of an attendance status to an attendance status of an object person during a first period;
a second model generation unit configured to generate a second prediction model by machine learning using second information, as learning data, the second information including at least attendance status information indicating an attendance status of each worker included in the similar worker group, measurement information including an amount of activity and sleeping hours of each worker, action record information indicating records of the time of an action performed by each worker for each action type, and psychological stress information indicating a level of psychological stress of each worker; and
a second prediction unit configured to predict a level of psychological stress of the object person from the first period onwards by inputting input data including at least attendance status information, measurement information, and action record information of the object person to the second prediction model.

5. The prediction system according to claim 4, wherein the attendance status information is information indicating at least one of the number of leaves taken, the number of annual paid leaves taken, average work start time, average work end time, and average working hours.

6. The prediction system according to claim 4, wherein the second model generation unit uses the second information, as the learning data, the second information including the attendance status information, the measurement information, the action record information, and the psychological stress information of each worker during the first period.

7. The prediction system according to claim 1 or 4, wherein the action record information includes information regarding how each worker spends time that is not working hours and during which a desired action is selectable.

8. A prediction method comprising:
a first extraction step of extracting a similar worker group from a plurality of workers on the basis of a degree of similarity of a working style to a working style of an object person during a first period;
a first model generation step of generating a first prediction model by machine learning using first information, as learning data, the first information including at least working style information indicating the working style of each worker included in the similar worker group, measurement information including an amount of activity and sleeping hours of each worker, action record information indicating records of the time of an action performed by each worker for each action type, and psychological stress information indicating a level of psychological stress of each worker; and
a first prediction step of predicting a level of psychological stress of the object person from the first period onwards by inputting input data including at least the working style information, the measurement information, and the action record information of the object person to the first prediction model.

9. A prediction method comprising:
a second extraction step of extracting a similar worker group from a plurality of workers on the basis of a degree of similarity of an attendance status to an attendance status of an object person during a first period;
a second model generation step of generating a second prediction model by machine learning using second information, as learning data, the second information including at least attendance status information indicating an attendance status of each worker included in the similar worker group, measurement information including an amount of activity and sleeping hours of each worker, action record information indicating records of the time of an action performed by each worker for each action type, and psychological stress information indicating a level of psychological stress of each worker; and
a second prediction step of predicting a level of psychological stress of the object person from the first period onwards by inputting input data including at least attendance status information, measurement information, and action record information of the object person to the second prediction model.

10. A program for causing a computer to function as the prediction system according to claim 1, the program causing the computer to function as the first extraction unit, the first model generation unit, and the first prediction unit.

11. A program for causing a computer to function as the prediction system according to claim 4, the program causing the computer to function as the second extraction unit, the second model generation unit, and the second prediction unit.
